(19) Europäisches Patentamt
European Patent Office
Office européen des brevets

(11) Veröffentlichungsnummer: **0 009 465**
**A1**

(12) # EUROPÄISCHE PATENTANMELDUNG

(21) Anmeldenummer: **79810094.7**

(22) Anmeldetag: **14.09.79**

(51) Int. Cl.³: **A 61 K 31/00,** C 07 D 401/14
// C07D401/04

(30) Priorität: **20.09.78 US 944057**

(43) Veröffentlichungstag der Anmeldung: **02.04.80**
**Patentblatt 80/7**

(84) Benannte Vertragsstaaten: **AT BE CH DE FR GB IT LU NL SE**

(71) Anmelder: **CIBA-GEIGY AG, Patentabteilung Postfach, CH-4002 Basel (CH)**

(72) Erfinder: **Werner, Lincoln Harvey, Dr., 94 Larned Road, Summit New Jersey, 07901 (US)**

(54) **N-(1-(4-Amino-2-chinazolinyl)-3- oder -4-piperidyl-lactame, Verfahren zu ihrer Herstellung, pharmazeutische Präparate enthaltend diese Verbindungen, sowie ihre therapeutische Verwendung.**

(57) Die Erfindung betrifft hypotensive und antihypertensive Verbindungen der Formel I

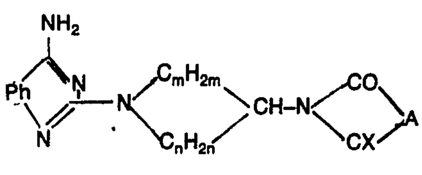

(I),

worin Ph einen 1,2-Phenylenrest bedeutet, der gegebenfalls durch höchstens 3 Substituenten ausgewählt von Niederalkyl und Niederalkoxy und einem Niederalkylendioxyd substituiert sein kann, jedes der Symbole m und n für eine ganze Zahl von 1 bis 3 steht, wobei m+n=4, X 2 Wasserstoffatome oder Oxo bedeutet, und A für Niederalkylen, 4 bis 7 Ringglieder enthaltendes Cycloalkylen, Cycloalkylniederalkylen und Spirocycloalkan-niederalkylen, HPh-Niederalkylen oder Ph steht, und ihre Säureadditionssalze. Sie können durch Umsetzung von 2-Halogen-chinazolinen mit entsprechenden Piperidinverbindungen hergestellt werden.

EP 0 009 465 A1

ACTORUM AG

0009465

CIBA-GEIGY AG
Basel (Schweiz)

4-12038/CGC 871/+

N-[1-(4-Amino-2-chinazolinyl)-3- oder -4-piperidyl-lactame, Verfahren zu ihrer Herstellung, pharmazeutische Präparate enthaltend diese Verbindungen, sowie ihre therapeutische Verwendung.

Gemäss dem US-Patent 3,511,836 sind 1-[4-Amino-2-chinazolinyl)-piperidine und ihre Derivate, welche in 4-Stellung des Piperidins Alkyl, Alkoxy, Hydroxy, Hydroxyalkyl, Phenyl, Benzyl und 4-Phenyl-4-carbonsäureniederalkylester enthalten, hypotensive Mittel. Es ist überraschend, dass die Verbindungen der vorliegenden Erfindung, welche in 3- oder 4-Stellung des Piperidins das Lactam-Stickstoffatom enthalten, überlegene hypotensive und antihypertensive Wirkungen, auf der Basis eines α-blockierenden Mechaninsmus, entfalten.

Die Erfindung betrifft neue N-[1-(4-Amino-2-chinazolinyl)-3- oder -4-piperidyl-lactame der allgemeinen Formel I

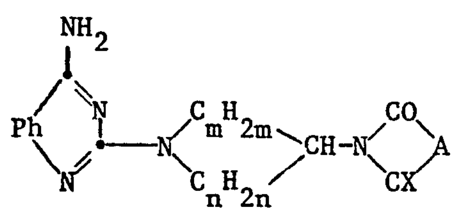

(I),

worin Ph einen 1,2-Phenylenrest bedeutet, der gegebenenfalls durch höchstens 3 Substituenten ausgewählt von Niederalkyl und Niederalkoxy und einem Niederalkylendioxy substituiert sein kann, jedes der Symbole m und n für eine ganze Zahl von 1 bis 3 steht, wobei $m + n = 4$, X 2 Wasserstoffatome oder Oxo bedeutet, und A für Niederalkylen, 4 bis 7 Ringglieder enthaltendes Cycloalkylen, Cycloalkylniederalkylen und Spirocycloalkan-niederalkylen, HPh-Niederalkylen oder Ph steht, sowie Säureadditionssalze, insbesondere therapeutisch verwendbare Säureadditionssalze dieser Verbindungen.

Eine im Chinazolin- und/oder Lactam-Teil der Verbindung vorhandene 1,2-Phenylengruppe Ph ist vorzugsweise mono-, di- oder trisubstituiert durch Niederalkyl, z.B. Methyl, Aethyl, n- oder i-Propyl oder -Butyl, oder Niederalkoxy, z.B. Methoxy, Aethoxy,n- oder i-Propoxy oder -Butoxy, oder monosubstituiert durch Niederalkylendioxy, z.B. Methylendioxy, 1,1- oder 1,2-Aethylendioxy. Die genannten Substituenten erscheinen vorzugsweise in 4,5- oder 4,5,6-Stellung der genannten Phenylengruppe.

Die Alkylengruppen $C_mH_{2m}$ und $C_nH_{2n}$, welche das Stickstoffatom von der Methingruppe durch 1 bis 3 Kohlenstoffatome trennen, bedeuten vorzugsweise jeweils Aethylen, aber auch Methylen und 1,3-Propylen.

Ein Niederalkylenrest "A" ist vorzugsweise Aethylen, 1,3-Propylen, 2,2-Di-(methyl, äthyl, n-propyl oder n-butyl)-äthylen oder 2,2-Di-(methyl, äthyl, n-propyl oder n-butyl)-1,3-propylen, 2,3- oder 1,4-Butylen.

Jeder der Cycloalkylen-, Cycloalkyl-niederalkylen oder Spirocycloalkan-niederalkylenreste weist 4 bis 7 Ringkohlenstoffatome auf und bedeutet z.B. 1,2-Cyclobutylen, 1,2- oder 1,3-(Cyclopentylen, Cyclohexylen oder Cycloheptylen); 1- oder 2-(Cyclopentyl oder Cyclohexyl)-äthylen oder 1- oder 2-(Cyclopentyl oder Cyclohexyl)-1,3-propylen; 1- oder 2-Spirocyclo-(butan, pentan oder hexan)-äthylen oder 1- oder 2-Spirocyclo-(butan, pentan oder hexan)-1,3-propylen.

Der Phenyl-niederalkylen- oder der 1,2-Phenylenrest, nämlich der HPh-Niederalkylenrest oder Ph-Rest "A" ist im Ring vorzugsweise unsubstituiert oder durch die oben genannten Alkyl, Alkoxy- oder Alkylendioxygruppen monosubstituiert. Das Symbol "A" bedeutet daher z.B. 1-Phenyl-äthylen, 2-Phenyl-1,3-propylen, 1- oder 2-(Tolyl oder Anisyl)-äthylen oder 1- oder 2-(Tolyl oder Anisyl)-1,3-propylen; 1,2-Phenylen, 3- oder 4-(Methyl oder Methoxy)-1,2-phenylen oder 4,5-Methylendioxy-1,2-phenylen.

Der Ausdruck "nieder" definiert in den oben oder nachfolgend genannten organischen Resten oder Verbindungen solche mit höchstens 8, vorzugsweise 4, insbesondere 1 oder 2 Kohlenstoffatomen.

Die basischen Verbindungen der Formel I bilden Säureadditionssalze. Bevorzugt sind die therapeutisch verwendbaren Säureadditionssalze, z.B. solche der weiter unter genannten Säuren.

Die Verbindungen der Erfindung zeigen wertvolle pharmakologische Eigenschaften, z.B. hypotensive, antihypertensive und Blutgefäss erweiternde Wirkungen. Diese pharmakologischen Eigenschaften können in Tierversuchen, vorzugsweise an Säugetieren, z.B. Ratten, Katzen oder Hunden, als Testobjekte, nachgewiesen werden. Die Tiere können normotensiv oder hypertensiv, z.B. genetisch oder renal hypertensive Ratten oder Hunde sein. Die neuen Verbindungen können ihnen enteral oder parenteral, vorzugsweise oral, subkutan, intravenös, intraperitoneal oder intraduodenal, z.B. durch Gelatine-Kapseln oder in Form von Stärke enthaltenden Suspensionen bzw. wässerigen Lösungen, verabreicht werden. Die verwendete Dosis kann in einem Bereich von ungefähr zwischen 0,1 und 100 mg/kg/Tag, vorzugsweise ungefähr 1 und 75 mg/kg/Tag, insbesondere zwischen 5 und 50 mg/kg/Tag liegen. Die Blutdrucksenkende Wirkung wird entweder direkt mit einem Katheter, der z.B. in die kaudale Arterie einer Ratte oder in die femurale Arterie eines Hundes eingeführt ist, und durch ein Uebertragungsinstrument den Blutdruck vor und nach der Verabreichung der Wirksubstanz in mm/Hg angibt, registriert, oder indirekt durch Sphygmomanometrie, z.B. am Rattenschwanz festgestellt. So sind z.B. das 1-[4-(4-Amino-6,7-dimethoxy-2-chinazolinyl)-4-piperidyl]-pyrrolidin-2,5-dion oder seine therapeutisch verwendbaren Salze als typische Vertreter von Verbindungen der Erfindung, in den genannten Versuchen stark antihypertensiv wirksam. Die Verbindungen der Erfindung können daher als antihypertensive Mittel, welche auf die Herzfrequenz keinen oder minimalen Einfluss haben, zur Behandlung oder Handhabung von essentieller oder

renaler Hypertension und/oder von kongestiven oder chronischen Herzstörungen in Säugern verwendet werden. Sie können auch als Zwischenprodukte zur Herstellung von anderen wertvollen, insbesondere von pharmakologisch wirksamen Verbindungen oder Präparaten eingesetzt werden.

Bevorzugte Verbindungen sind diejenigen der Formel I, worin Ph einen 1,2-Phenylenrest bedeutet, der gegebenenfalls durch höchstens 3 Substituenten ausgewählt von Alkyl oder Alkoxy mit höchstens 4 Kohlenstoffatomen und einem Alkylendioxy mit höchstens 2 Kohlenstoffatomen substituiert sein kann, jedes der Symbole m und n für eine ganze Zahl von 1 bis 3 steht, wobei m + n = 4, X zwei Wasserstoffatome oder Oxo bedeutet, und A für Niederalkylen, 4 bis 7 Ringglieder enthaltendes Cycloalkylen, Cycloalkyl-niederalkylen, Spirocycloalkan-niederalkylen, HPh-Niederalkylen oder Ph steht, und ihre therapeutisch verwendbaren Säureadditionssalze.

Bevorzugt sind weiter Verbindungen der Formel I, worin Ph 1,2-Phenylen, Mono-, Di- oder Tri-(alkyl oder alkoxy)-1,2-phenylen oder Alkylendioxy-1,2-phenylen bedeutet, in welchen Alkyl, Alkoxy oder Alkylen höchstens 2 Kohlenstoffatome enthält, jede der Gruppen $C_mH_{2m}$ und $C_nH_{2n}$ für Aethylen steht, X zwei Wasserstoffatome oder Oxo bedeutet, und A für Niederalkylen, 5 oder 6 Ringglieder enthaltendes 1,2-Cycloalkylen oder (Cycloalkyl, Spirocycloalkan oder Phenyl)-alkylen steht, wobei jeder der letztgenannten drei Reste eine Summe von höchstens 8 Kohlenstoffatomen enthält, und ihre therapeutisch verwendbaren Säureadditionssalze.

Besonders hervorzuheben sind Verbindungen der allgemeinen Formel II

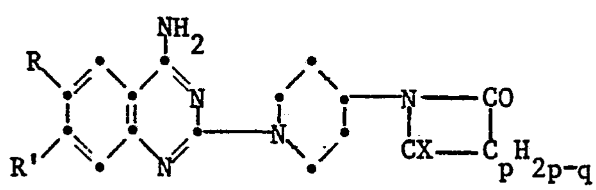

(II),

worin jedes der Symbole R und R' Methoxy oder zusammen Methylendioxy bedeutet, X für zwei Wasserstoffatome oder Oxo steht, $C_pH_{2p-q}$ Alkylen, Phenyl-alkylen oder Spirocycloalkan-alkylen bedeutet, wobei p eine ganze Zahl von 2 bis 8 ist, q die Zahl 0,2 oder 8 bedeutet, und $2p-q$ positiv ist, und ihre therapeutisch verwendbaren Säureadditionssalze.

Bevorzugt sind weiter Verbindungen der Formel II, worin jedes der Symbole R und R' Methoxy bedeutet, X für Oxo steht und das Symbol $C_pH_{2p-q}$ Aethylen, Phenyläthylen oder 1,3-Propylen bedeutet, und ihre therapeutisch verwendbaren Säureadditionssalze.

Die Verbindungen der vorliegenden Erfindung werden nach an sich bekannten Methoden, z.B. dadurch hergestellt, dass man Verbindungen der Formeln III und IV

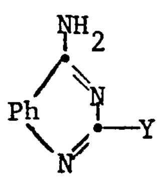

(III)

+

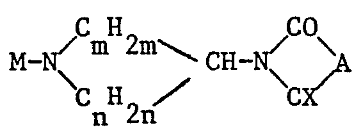

(IV)

kondensiert, worin Y Halogen oder Niederalkythio bedeutet und M für Wasserstoff oder ein Alkalimetallatom steht, und, wenn erwünscht, eine erhaltene Verbindung der Formel I in eine andere erfindungsgemässe Verbindung umwandelt.

Das Halogenatom Y ist vorzugsweise Chlor oder Brom. Eine Niederalkylthiogruppe Y ist in erster Linie Methylthio. Das Alkalimetallatom M ist vorzugsweise Natrium oder Kalium.

Die genannte Kondensation wird bei Temperaturen über Zimmertemperatur, z.B. zwischen ungefähr 100 und ungefähr 200° und/oder in Gegenwart von Kondensationsmitteln, welche die gebildeten Säuren binden, durchgeführt. Solche Mittel sind Alkalimetall-carbonate oder

-hydrogencarbonate, oder tertiäre Amine, z.B. Tri-niederalkylamine, Pyridine bzw. niederalkylierte Pyridine.

Erhaltene freie Basen können in entsprechende Säureadditionssalze, vorzugsweise unter Verwendung von Säuren, welche therapeutisch verwendbare Säureadditionssalze ergeben, oder mit entsprechenden Anionenaustauschern, umgewandelt werden. Erhaltene Säureadditionssalze können in die entsprechenden freien Basen z.B. durch Behandlung mit einer Base, wie einem Metallhydroxyd, basischem Salz, Ammoniak, Amin oder einem Kationenaustauscher, z.B. einem Alkalimetallhydroxyd oder -carbonat, übergeführt werden. Säuren, die therapeutisch verwendbare Säureadditionssalze ergeben, sind z.B. anorganische Säuren, wie Halogenwasserstoffsäuren, z.B. Chlorwasserstoff- oder Bromwasserstoffsäure, oder Schwefel-, Phosphor-, Salpeter- oder Perchlorsäure; oder organische Säuren, wie aliphatische oder aromatische Carbon- und Sulfonsäuren, z.B. Ameisen-, Essig-, Propion-, Bernstein-, Glykol-, Milch-, Aepfel-, Wein-, Zitronen-, Malein-, Hydroxymalein-, Brenztrauben-, Phenylessig-, Benzoe-, 4-Aminobenzoe-, Anthranil-, 4-Hydroxybenzoe-, Salicyl-, 4-Aminosalicyl-, Pamoe-, Nikotin-, Methansulfon-, Aethansulfon-, Hydroxyäthansulfon-, Aethylensulfon-, Halogenbenzolsulfon-, Toluolsulfon-, Naphthalinsulfon-, Sulfanil- oder Cyclohexylsulfaminsäure; oder die Ascorbinsäure.

Diese oder andere Salze, z.B. die Pikrate, können auch in der Reinigung von freien Basen verwendet werden. Die Basen werden in ihre Salze übergeführt, die Salze abgetrennt und die Basen aus den Salzen freigesetzt. Infolge der engen Beziehungen zwischen den neuen Verbindungen in freier Form und in Form ihrer Salze sind im vorausgegangenenen und nachfolgend unter freien Verbindungen und Salzen sinn- und zweckgemäss gegebenenfalls auch die entsprechenden Salze bzw. freien Verbindungen zu verstehen.

Die Ausgangsstoffe der Formeln III und IV sind bekannt, oder wenn neu, können sie nach an sich bekannten Methoden, z.B. wie in

den Beispielen illustriert hergestellt werden. Bekannte Ausgangsstoffe und ihre Vorstufen sind auch z.B. in den US-Patenten 3,511,836; 3,769,286 und im US-Patent 4,000,287 des Anmelders, aber auch in den in diesen angegebenen Referenzen, beschrieben.

Endprodukte der Formel I, welche Isomerengemische sind, können nach an sich bekannten Methoden, z.B. durch fraktionierte Destillation, Kristallisation und/oder Chromatographie, in die einzelnen Isomeren getrennt werden. Racemische Produkte können in die optischen Antipoden, z.B. durch Trennung ihrer diastereomeren Salze, z.B. durch fraktionierte Kristallisation der d- oder l-Tatrate, getrennt werden.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensationsmitteln oder anderen genannten Mitteln, und/oder in einer inerten Atmosphäre, unter Kühlung, bei Zimmertemperatur oder bei erhöhten Temperaturen, vorzugsweise beim Siedepunkt des verwendeten Lösungsmittels, bei normalem oder erhöhtem Druck durchgeführt.

Die Erfindung betrifft ebenfalls Abänderungen des vorliegenden Verfahrens, wonach ein auf irgendeiner Stufe des Verfahrens erhältliches Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangstoff in Form eines Salzes oder eines optisch reinen Antipoden verwendet wird.

Beim Verfahren der vorliegenden Erfindung werden vorzugsweise solche Ausgangstoffe verwendet, welche zu den eingangs als besonders wertvoll geschilderten Verbindungen, insbesondere solchen der Formel II führen.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z.B. Laktose, Dextrose, Rohrzucker, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, Enzyme der Bindemittel und/oder Brausemischungen, oder Adsorptionsmittel, Farbstoffe, Geschmackstoffe und Süssmittel. Injizierbare oder inhalierbare Präparate sind vorzugsweise isotonische wässerige Lösungen oder Suspensionen, und Suppositorien in erster Linie Fettemulsionen oder -suspensionen. Die pharmakologischen Präparate können sterilisiert sein und/oder Hilfstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0,1% bis etwa 75%, insbesondere von etwa 1% bis etwa 50% des Aktivstoffes.

Die folgenden Beispiele dienen zur Illustration der Erfindung. Temperaturen werden in Celsiusgraden angegeben, und die Angaben über Teile betreffen Gewichtsteile. Wenn nicht anders definiert, wird das Eindampfen von Lösungsmitteln unter vermindertem Druck, z.B. zwischen ungefähr 1 und 100 mm/Hg, durchgeführt.

Beispiel 1 Ein Gemisch von 8,66 g 4-Amino-2-chlor-6,7-dimethoxy-chinazolin, 6,6 g 1-(4-Piperidyl)-pyrrolidin-2,5-dion, 9,4 g Diisopropyläthylamin und 100 ml Dimethylformamid wird in einer Stickstoffatmosphäre bei 150° 8 Stunden gerührt. Das Reaktionsgemisch wird eingedampft, der Rückstand mit wässeriger Natriumcarbonatlösung trituriert und mit Essigsäureäthylester extrahiert. Der Extrakt wird gewaschen, getrocknet, eingedampft und der Rückstand aus Aethanol umkristallisiert. Die Kristalle werden in einem Gemisch von Aethanol-Aceton aufgelöst und die Lösung wird mit Methansulfonsäure neutralisiert. Der erhaltene Niederschlag wird abfiltriert, getrocknet und aus wässerigem Aethanol umkristallisiert. Man erhält das 1-[1-(4-Amino-6,7-dimethoxy-2-chinazolinyl)-4-piperidyl]-pyrrolidin-2,5-dion-monomethansulfonat der Formel

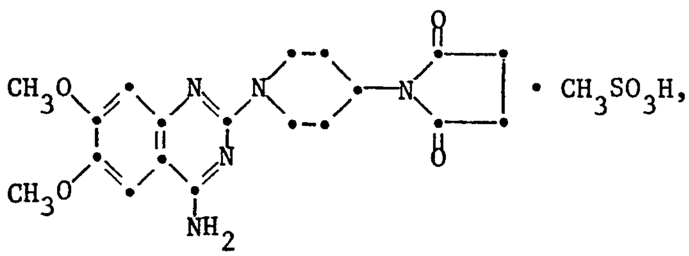

welches bei 331° unter Zersetzung schmilzt.

Der Ausgangstoff wird wie folgt hergestellt: Ein Gemisch von 20 g Bernsteinsäure-anhydrid, 19 g 4-Aminopyridin und 450 ml Xylol wird unter Rühren 24 Stunden gerührt. Das Reaktionsgemisch wird auf Zimmertemperatur abgekühlt, das feste Material abgetrennt und dreimal mit 200 ml Methylenchlorid unter Rückfluss extrahiert. Die vereinigten Extrakte werden eingedampft und der Rückstand aus Aethanol umkristallisiert. Man erhält das 1-(4-Pyridyl)-pyrrolidin-2,5-dion.

Man hydriert 11,36 g der letztgenannten Verbindung bei 60° in einer minimalen Menge Eisessig, über 8 g 10%-igem Palladium-auf-Kohle Katalysator bei 2,7 Atmosphären. Das Reaktionsgemisch wird filtriert und eingedampft. Der Rückstand wird mit wässeriger Natriumcarbonatlösung basisch gemacht, mit Chloroform extrahiert und der Extrakt eingedampft. Man erhält das 1-(4-Piperidyl)-pyrrolidin-2,5-dion, welches bei 133-134° schmilzt.

Beispiel 2 Ein Gemisch von 6,7 g 4-Amino-2-chlor-6,7-dimethoxy-chinazolin, 5,5 g 1-(4-Piperidyl)-piperidin-2,6-dion, 7,2 g Diisopropyläthylamin und 75 ml Dimethylformamid wird in einer Stickstoffatmosphäre 8 Stunden bei 150° gerührt. Das Reaktionsgemisch wird auf Zimmertemperatur gekühlt, filtriert und das Filtrat eingedampft. Der Rückstand wird mit Aethanol trituriert, wieder filtriert und die beiden Niederschläge vereinigt. Dieses feste Material wird in wässeriger Natriumcarbonatlösung aufgenommen, das Gemisch mit Essigsäureäthylester extrahiert, der Extrakt mit Wasser gewaschen, getrocknet und eingedampft. Der Rückstand wird in warmem Aethanol aufgenommen, die Lösung mit Methansulfonsäure angesäuert, der erhaltene Niederschlag abfiltriert und aus wässerigem Aethanol umkristallisiert. Man erhält das 1-[1-(4-Amino-6,7-dimethoxy-2-chinazolinyl)-4-piperidyl]-piperidin-2,6-dion-monomethansulfonat, welches bei 333-334° schmilzt.

Der Ausgangstoff wird wie folgt hergestellt: Ein Gemisch von 34,2 g Glutarsäureanhydrid, 28,2 g 4-Amino-pyridin und 650 ml Xylol wird unter Rühren 6 Stunden unter Rückfluss gekocht. Das Reaktionsgemisch wird auf Zimmertemperatur abgekühlt, der Niederschlag abfiltriert und zweimal mit 200 ml Chloroform unter Rückfluss extrahiert. Die vereinigten Extrakte werden eingedampft und der Rückstand wird zweimal aus Essigsäureäthylester umkristallisiert. Man erhält das 1-(4-Pyridyl)-piperidin-2,6-dion,welches bei 153-155° schmilzt.

Man hydriert 8,1 g der letztgenannten Verbindung in 100 ml Eisessig über 6,3 g 10%-igem Palladium-auf-Kohle Katalysator bei 60° und 2,9 Atmosphären. Das Reaktionsgemisch wird abfiltriert, das Filtrat eingedampft und der Rückstand mit wässeriger Natriumcarbonatlösung behandelt. Das Gemisch wird mehrmals mit Methylenchlorid extrahiert und die vereinigten Extrakte werden eingedampft. Man erhält

das 1-(4-Piperidyl)-piperidin-2,6-dion als solvatisiertes Material mit niedrigem Schmelzpunkt.

Beispiel 3 Ein Gemisch von 4,79 g 4-Amino-2-chlor-6,7-dimethoxy-chinazolin, 4,49 g 4,4-Dimethyl-1-(4-piperidyl)-piperidin-2,6-dion, 4,24 g wasserfreiem Natriumcarbonat und 75 ml Dimethylformamid wird in einer Stickstoffatmosphäre 6 Stunden bei 150° gerührt. Das Reaktionsgemisch wird abfiltriert, das Filtrat eingedampft, der Rückstand in Essigsäureäthylester gelöst und die Lösung mit wässeriger Natriumcarbonatlösung und Wasser gewaschen. Die Lösung wird getrocknet, eingedampft, der Rückstand mit Isopropanol trituriert, filtriert und das feste Material verworfen. Das Filtrat wird mit Chlorwasserstoff stark sauer gemacht, der Niederschlag abfiltriert und mit 175 ml siedendem Methanol trituriert. Man erhält das 1-[1-(4-Amino-6,7-dimethoxy-2-chinazolinyl)-4-piperidyl]-4,4-dimethyl-piperidin-2,6-dion-monohydrochlorid, welches unter Zersetzung bei 279-280° schmilzt.

Der Ausgangsstoff wird wie folgt hergestellt: Ein Gemisch von 19,3 g 3,3-Dimethylglutarsäure-anhydrid, 12,55 g 4-Amino-pyridin und 350 ml Xylol wird unter Verwendung eines Wasserabscheiders 60 Stunden unter Rühren unter Rückfluss gekocht. Die heisse Xylollösung wird von sirupartigem Rückstand dekantiert, auf Zimmertemperatur gekühlt, filtriert und der Rückstand aus Aethanol umkristallisiert. Man erhält das 4,4-Dimethyl-1-(4-pyridyl)-piperidin-2,6-dion, welches bei 225-226° schmilzt.

Man löst 20 g der letztgenannten Verbindung in 200 ml Eisessig bei 60° und hydriert sie über 15 g 10%-igem Palladium-auf-Kohle Katalysator bei 3,1 Atmosphären. Das Reaktionsgemisch wird filtriert, eingedampft, der Rückstand unter Kühlen in 6-normaler wässeriger Natriumhydroxydlösung aufgenommen und das Gemisch mit Methylenchlorid extrahiert. Der Extrakt wird eingedampft und der Rückstand aus einem Gemisch von Benzol und Hexan umkristallisiert. Man erhält das 4,4-Dimethyl-1-(4-piperidyl)-1-piperidin-2,6-dion, welches bei 162-163° schmilzt.

Beispiel 4 Ein Gemisch von 70 g 4-Amino-2-chlor-6,7-dimethoxychinazolin, 73,2 g 8-(4-Piperidyl)-8-azaspiro[4,5]decan-7,9-dion, 62,1 g wasserfreiem Natriumcharbonat und 800 ml Dimethylformamid wird in einer Stickstoffatmosphäre bei 135° 8 Stunden gerührt. Das Reaktionsgemisch lässt man auf Zimmertemperatur abkühlen, filtriert es und dampft das Filtrat ein. Der Rückstand wird unter Rühren und Kühlen in 300 ml Wasser gegossen, das Gemisch mit 1400 ml Wasser verdünnt und filtriert. Das feste Material wird mit 1600 ml Essigsäureäthylester und 10 ml gesättigter wässeriger Natriumcarbonatlösung trituriert. Die organische Schicht wird abgetrennt, getrocknet, eingedampft und der Rückstand in 1000 ml Essigsäureäthylester aufgelöst. Die Lösung wird mit Chlorwasserstoff angesäuert, der Niederschlag abfiltriert und aus wässerigem Methanol umkristallisiert. Man erhält das 8-[1-(4-Amino-6,7-dimethoxy-2-chinazolinyl)-4-piperidyl]-8-aza spiro[4,5]decan-7,9-dion-hydrochlorid, welches unter Zersetzung bei 277-279° schmilzt.

Man suspendiert 5 g der letztgenannten Verbindung in 100 ml Wasser und 10 ml 6-normaler wässeriger Natriumhydroxydlösung und extrahiert das Gemisch mit Chloroform. Der Extrakt wird mit Wasser gewaschen, getrocknet, eingedampft und der Rückstand in 50 ml Aethanol unter Erwärmen gelöst. Die Lösung wird mit 0,94 g Methansulfonsäure versetzt, der Niederschlag abgetrennt und aus wässerigem Aethanol umkristallisiert. Man erhält das ensprechende Monomethansulfonat, welches bei 307-308° schmilzt.

Der Ausgangsstoff wird wie folgt hergestellt: Ein Gemisch von 100 g 3,3-Tetramethylen-glutarsäure-anhydrid, 57 g 4-Amino-pyridin und 1500 ml Xylol wird gerührt und unter Verwendung eines Wasserabscheiders 3 Tage unter Rückfluss gekocht. Das Reaktionsgemisch wird etwas abgekühlt, die Xylollösung von einer geringen Menge eines öligen Materials dekantiert und im Eisbad gekühlt. Der erhaltene Niederschlag wird abgetrennt, in 1500 ml siedendem Aethanol gelöst, die Lösung auf 5° gekühlt, filtriert und der Rückstand getrocknet. Man

erhält das 8-(4-Pyridyl)-8-azaspiro[4,5]decan-7,8-dion, welches bei 208-211° schmilzt.

Eine Lösung von 58 g der letztgenannten Verbindung in 580 ml Eisessig wird über 45 g 10%-igem Palladium-auf-Kohle Katalysator bei Zimmertemperatur und 3,1 Atmosphären hydriert. Nach der Aufnahme der theoretischen Menge Wasserstoff wird das Gemisch filtriert und eingedampft. Der Rückstand wird mit 6-normaler wässeriger Natriumhydroxydlösung basisch gemacht und das Gemisch mit Chloroform extrahiert. Der Extrakt wird mit gesättigter wässeriger Natriumchloridlösung gewaschen, getrocknet, eingedampft und der Rückstand in 325 ml heissem Toluol aufgelöst. Die Lösung wird filtriert, mit 400 ml Hexan verdünnt, abgekühlt und filtriert. Man erhält das 8-(4-Piperidyl)-8-azaspiro[4,5]decan-7,9-dion, welches bei 141-142° schmilzt.

Beispiel 5  Ein Gemisch von 5,39 g 4-Amino-2-chlor-6,7,8-trimethoxychinazolin, 5,25 g 8-(4-Piperidyl)-8-azaspiro[4,5]decan-7,9-dion, 4,24 g wasserfreiem Natriumcarbonat und 75 ml Dimethylformamid wird 8 Stunden bei 150° gerührt. Das Reaktionsgemisch wird nach Abkühlen auf Zimmertemperatur filtriert, das Filtrat eingedampft, der Rückstand in Essigsäureäthylester gelöst und die Lösung mit wässeriger Natriumhydrogencarbonatlösung und Wasser gewaschen. Die Lösung wird getrocknet, eingedampft, der Rückstand in 75 ml Essigsäureäthylester gelöst und kristallisieren gelassen. Die Kristalle werden in 50 ml heissem Isopropanol gelöst und die Lösung mit Chlorwasserstoff auf den pH-Wert 1 eingestellt. Der erhaltene Niederschlag wird abfiltriert. Man erhält das 8-[1-(4-Amino-6,7,8-trimethoxy-2-chinazolinyl)-4-piperidyl]-8-azaspiro[4,5]decan-7,9-dion-monohydrochlorid, welches bei 225-227° schmilzt.

Beispiel 6  Ein Gemisch von 2,39 g 4-Amino-2-chlor-6,7-dimethoxychinazolin, 2,64 g 3-(4-Piperidyl)-3-azaspiro[5,5]undecan-2,4-dion, 2,1 g wasserfreiem Natriumcarbonat und 25 ml Dimethylformamid wird in einer Stickstoffatmosphäre 10 Stunden bei 150° gerührt. Das Reaktionsgemisch wird filtriert, das Filtrat eingedampft und der Rückstand in Methylenchlorid aufgelöst. Die Lösung wird mit wässeriger Natriumcarbonatlösung gewaschen, getrocknet, filtriert und eingedampft. Der Rückstand wird mit 50 ml Isopropanol trituriert, das zurückgebliebene Material in Isopropanol aufgenommen und mit Chlorwasserstoff angesäuert. Man erhält das 3-[1-(4-Amino-6,7-dimethoxy-2-chinazolinyl)-4-piperidyl]-3-azaspiro[5,5]undecan-2,4-dion-monohydrochlorid, welches bei 290° unter Zersetzung schmilzt.

Der Ausgangsstoff wird wie folgt hergestellt: Ein Gemisch von 25 g 1,1-Cyclohexan-diessigsäure-anhydrid, 12,92 g 4-Amino-pyridin und 400 ml Xylol wird unter Verwendung eines Wasserabscheiders 25 Stunden unter Rückfluss gekocht. Die heisse obenstehende Xylollösung wird von einem öligen Niederschlag (der verworfen wird) dekantiert, abgekühlt, filtriert und der Rückstand aus Aethanol umkristallisiert. Man erhält das 3-(4-Pyridyl)-3-azaspiro[5,5]undecan-2,4-dion, welches bei 213-215° schmilzt.

Man löst 13,8 g der letztgenannten Verbindung in 150 ml Eisessig und hydriert sie über 10 g 10%-igem Palladium-auf-Kohle Katalysator bei 50-60° und 1,7 Atmosphären. Das Gemisch wird filtriert und der Rückstand gemäss Beispiel 4 aufgearbeitet. Man erhält das 3-(4-Piperidyl)-3-azaspiro[5,5]undecan-2,4-dion, welches nach Umkristallisation aus Benzol-Hexan bei 148-149° schmilzt.

<u>Beispiel 7</u> Ein Gemisch von 5,72 g 4-Amino-2-chlor-6,7-dimethoxychinazolin, 5,65 g 2-(4-Piperidyl)-2-azaspiro[4,4]nonan-1,3-dion, 6,16 g Diisopropyläthylamin und 75 ml Dimethylformamid wird in einer Stickstoffatmosphäre 8 Stunden bei 150° gerührt. Das Reaktionsgemisch wird eingedampft, der Rückstand zwischen Essigsäureäthylester und wässeriger Natriumcarbonatlösung verteilt, die organische Lösung mit Wasser gewaschen, getrocknet und eingedampft. Der Rückstand wird in Aethanol aufgenommen und mit Methansulfonsäure auf den pH-Wert 1 eingestellt. Der erhaltene Niederschlag wird abfiltriert und

zuerst aus Methanol und dann aus wässerigem Aethanol umkristallisiert. Man erhält das 2-[1-(4-Amino-6,7-dimethoxy-2-chinazolinyl)-4-piperidyl]-2-azaspiro[4,4]nonan-1,3-dion-monomethansulfonat, welches bei 278-280° schmilzt.

Der Ausgangsstoff wird wie folgt hergestellt: Ein Gemisch von 21,8 g Tetramethylen-bernsteinsäure-anhydrid (hergestellt aus der entsprechenden Dicarbonsäure durch Kochen mit Essigsäure-anhydrid unter Rückfluss), 12,2 g 4-Amino-pyridin und 300 ml Xylol wird unter Verwendung eines Wasserabscheiders 9 Stunden unter Rückfluss gekocht. Die heisse Lösung wird filtriert, auf 5° gekühlt und der Rückstand aus Aethanol umkristallisiert. Man erhält das 2-(4-Pyridyl)-2-aza-spiro[4,4]nonan-1,3-dion, welches bei 100-103° schmilzt.

Man hydriert 16,76 g der letztgenannten Verbindung über 12 g 10%-igem Palladium-auf-Kohle Katalysator in 180 ml Eisessig bei 60° und 3,2 Atmosphären. Das Gemisch wird filtriert, eingedampft, der Rückstand mit wässeriger Natriumcarbonatlösung basisch gemacht und mehrmals mit Chloroform extrahiert. Die Extrakte werden getrocknet und konzentriert. Man erhält das 2-(4-Piperidyl)-2-azaspiro[4,4]nonan-1,3-dion, welches bei 119-120° schmilzt.

Beispiel 8 Ein Gemisch von 4,8 g 4-Amino-2-chlor-6,7-dimethoxy-chinazolin, 5.17 g 1-(4-Piperidyl)-3-phenyl-pyrrolidin-2,5-dion, 5,2 g Diisopropyläthylamin und 75 ml Dimethylformamid wird in einer Stickstoffatmosphäre 8 Stunden bei 150° gerührt. Das Reaktionsgemisch wird eingedampft, der Rückstand zwischen Essigsäureäthylester und wässeriger Natriumcarbonatlösung verteilt, die organische Schicht abgetrennt, getrocknet und eingedampft. Der Rückstand wird in warmem Aethanol gelöst und die Lösung mit Methansulfonsäure auf den pH-Wert 1 eingestellt. Der nach Kühlen erhaltene Niederschlag wird abgetrennt und aus wässerigem Aethanol umkristallisiert. Man erhält das 1-[1-(4-Amino-6,7-dimethoxy-2-chinazolinyl)-4-piperidyl]-3-phenyl-pyrrolidin-2,5-dion-monomethansulfonat, welches unter Zersetzung bei 239-244° schmilzt.

Der Ausgangsstoff wird wie folgt hergestellt: Ein Gemisch von 20,2 g Phenylbernsteinsäure-anhydrid, 10,7 g 4-Amino-pyridin und 300 ml Xylol wird unter Verwendung eines Wasserabscheiders 6 Stunden unter Rückfluss gekocht. Die heisse Lösung wird von etwas gummiartigem Niederschlag dekantiert. Nach Abkühlen erhält man einen Niederschlag, der abfiltriert und aus Aethanol umkristallisiert wird. Man erhält das 3-Phenyl-1-(4-pyridyl)-pyrrolidin-2,5-dion, welches bei 146-147° schmilzt.

Man hydriert 20,6 g der letztgenannten Verbindung in 200 ml Eisessig über 14 g 10%-igem Palladium-auf-Kohle Katalysator bei 60° und 3 Atmosphären. Das Gemisch wird abfiltriert, eingedampft und der Rückstand in 75 ml Wasser aufgelöst. Die Lösung wird mit Natriumcarbonat basisch gemacht und mehrmals mit Essigsäureäthylester extrahiert. Der Extrakt wird mit wässeriger Natriumcarbonatlösung gewaschen, getrocknet und eingedampft. Der Rückstand wird aus einem Gemisch von Toluol-Hexan umkristallisiert. Man erhält das 1-(4-Piperidyl)-3-phenyl-pyrrolidin-2,5-dion, welches bei 119-124° schmilzt.

<u>Beispiel 9</u> Ein Gemisch von 5,39 g 4-Amino-2-chlor-6,7,8-trimethoxychinazolin, 4,32 g 2-(4-Piperidyl)-isoindolin-1-on, 4,24 g wasserfreiem Natriumcarbonat und 75 ml Dimethylformamid wird in einer Stickstoffatmosphäre 16 Stunden bei 125-130° gerührt. Das Reaktionsgemisch wird filtriert, das Filtrat eingedampft, der Rückstand in Methylenchlorid aufgenommen und die Lösung mit wässeriger Natriumhydrogencarbonatlösung gewaschen. Die organische Schicht wird abgetrennt, getrocknet und eingedampft. Der Rückstand wird in unter Rückfluss kochendem Isopropanol suspendiert und mit Chlorwasserstoff auf den pH-Wert 1 eingestellt. Der erhaltene Niederschlag wird abfiltriert und mit warmem Aethanol trituriert. Man erhält das 2-[1-(4-Amino-6,7,8-trimethoxy-2-chinazolinyl)-4-piperidyl]-isoindolin-1-on-monohydrochlorid, welches unter Zersetzung bei 234° schmilzt.

In analoger Weise erhält man durch Umsetzung von 2,4 g 4-Amino-2-chlor-6,7-dimethoxy-chinazolin, 2,2 g 2-(4-Piperidyl)-isoindolin-1-on und 2 g wasserfreiem Natriumcarbonat in 20 ml Dimethylformamid das 2-[1-(4-Amino-6,7-dimethoxy-2-chinazolinyl)-4-piperidyl]-isoindolin-1-on-monohydrochlorid, welches unter Zersetzung bei 288-289° schmilzt.

Der Ausgangsstoff wird wie folgt hergestellt: Ein Gemisch von 2-Formyl-benzoesäure, 85,2 g 4-Amino-pyridin und 2800 ml Toluol wird unter Verwendung eines Wasserabscheiders 2,5 Stunden gerührt und unter Rückfluss gekocht und das Rühren wird bei Zimmertemperatur über Nacht fortgesetzt. Das Reaktionsgemisch wird filtriert und der Rückstand mit Toluol gewaschen. Man erhält das 1-(4-Pyridylamino)-3-oxo-phthalan, welches bei 215-220° schmilzt. [In analoger Weise wird das 1-(3-Pyridylamino)-3-oxo-phthalan ausgehend von 3-Amino-pyridin, erhalten. F. 150-155°].

Eine Suspension von 278 g der bei 215-220° schmelzenden Verbindung in 4700 ml wasserfreiem Aethanol wird unter Rühren bei 18° mit 96 g Natriumborhydrid innerhalbe 105 Minuten protionenweise versetzt. Das Rühren wird über Nach bei Zimmertemperatur fortgesetzt, das Gemisch filtriert, das Filtrat zu einem Volumen von 1200 ml konzentriert, gekühlt und der erhaltene Niederschlag abgetrennt. Man erhält die 2-(4-Pyridylaminomethyl)-benzoesäure, welche bei über 250° schmilzt.

Man gibt 180 g der letztgenannten Verbindung zu 1400 ml konzentrierter Schwefelsäure innerhalb 40 Minuten unter Rühren und lässt die Temperatur auf ungefähr 67° steigen. Das Gemisch wird eine Stunde bei ungefähr 95° gerührt, auf 25° gekühlt und langsam auf 4000 g Eis gegossen. Das Gemisch wird mit ungefähr 4500 ml wässerigem Ammoniak neutralisiert, der erhaltene Niederschlag abfiltriert und in 2300 ml Isopropanol und 600 ml Chloroform aufgenommen. Das Gemisch wird 30 Minuten unter Rückfluss gekocht, heiss filtriert, das Filtrat

gekühlt und der erhaltene Niederschlag abgetrennt. Man erhält das 2-(4-Pyridyl)-isoindolin-1-on.

Ein Gemisch von 30 g der letztgenannten Verbindung, 400 ml Eisessig und 30 g 10%-igem Palladium-auf-Kohle Katalysator wird bei ungefähr 65° und 3 Atmosphären bis zur Aufnahme der theoretischen Menge Wasserstoff hydriert. Das Gemisch wird auf Zimmertemperatur gekühlt, filtriert und eingedampft. Der Rückstand wird in 6-normaler wässeriger Natriumhydroxydlösung aufgenommen, das Gemisch mit Chloroform extrahiert, der Extrakt mit gesättigter wässeriger Natriumchloridlösung gewaschen, getrocknet und eingedampft. Man erhält das 2-(4-Piperidyl)-isoindolin-1-on, welches bei 144-146° schmilzt.

Beispiel 10 Ein Gemisch von 4,4 g 4-Cyclohexyl-1-(4-piperidyl)-piperidin-2,6-dion, 3,41 g 4-Amino-2-chlor-6,7-dimethoxy-chinazolin und 100 ml Isoamylalkohol wird unter Rühren 20 Stunden unter Rückfluss gekocht. Das Reaktionsgemisch wird 18 Stunden bei Zimmertemperatur stehen gelassen, filtriert, der Rückstand mit Isoamylalkohol und Diäthyläther gewaschen, getrocknet und in wässeriger Natriumcarbonatlösung aufgelöst. Die Lösung wird mit Methylenchlorid extrahiert, der Extrakt eingedampft und 7 g des Rückstands werden in 75 ml Aethanol aufgelöst. Die Lösung wird zuerst mit 1,4 g Methansulfonsäure, dann mit 125 ml Aethanol versetzt, der erhaltene Niederschlag abfiltriert und aus wässerigem Aethanol umkristallisiert. Man erhält das 1-[1-(4-Amino-6,7-dimethoxy-2-chinazolinyl)-4-piperidyl]-4-cyclohexyl-piperidin-2,6-dion-monomethansulfonat, welches bei 277-279° schmilzt.

Der Ausgangsstoff wird wie folgt hergestellt: Ein Gemisch von 22,4 g 3-(p-Chlorphenyl)-glutarsäure-anhydrid, 10,3 g 4-Aminopyridin, 350 ml Xylol und 1,5 g Methansulfonsäure wird unter Verwendung eines Wasserabscheiders 4 Tage gerührt und unter Rückfluss gekocht. Nach dem ersten Tag wird das Reaktionsgemisch mit 0,5 g

Methansulfonsäure versetzt und am Ende die heisse Lösung von etwas gummiartigem Material dekantiert. Die nach Kühlen erhaltenen Kristalle werden abfiltriert, mit heissem Aethanol trituriert, abgekühlt, wieder filtriert und getrocknet. Man erhält das 4-(p-Chlorphenyl)-1-(4-pyridyl)-piperidin-2,6-dion, welches bei 204-206° schmilzt.

Ein Gemisch von 11 g der letztgenannten Verbindung, 5,5 g 10%-igem Palladium-auf-Kohle Katalysator und 150 ml Eisessig wird 10 Stunden bei 120-140°C und 3 Atmosphären hydriert. Die Lösung wird nach Abkühlen abfiltriert, eingedampft und der Rückstand in einer minimalen Menge Wasser aufgelöst. Die Lösung wird mit 2-normaler wässeriger Natriumhydroxydlösung basisch gemacht und mit Methylenchlorid extrahiert. Der Extrakt wird mit Wasser gewaschen und eingedampft. Man erhält das 4-Cyclohexyl-1-(4-piperidyl)-piperidin-2,6-dion, dessen Hydrochlorid bei 284-285° unter Zersetzung schmilzt.

Beispiel 11 Ein Gemisch von 4,57 g 4-Amino-2-chlor-6,7-dimethoxy-chinazolin, 5,95 g 3-(p-Methoxyphenyl)-1-(4-piperidyl)-pyrrolidin-2,5-dion und 125 ml Isoamylalkohol wird 24 Stunden unter Rückfluss gekocht. Das Reaktionsgemisch wird zwei Tage bei Zimmertemperatur stehen gelassen, filtriert, der Rückstand mit Isoamylalkohol und Diäthyläther gewaschen, getrocknet und in wässeriger Natriumcarbonatlösung gelöst. Die Lösung wird mit Methylenchlorid extrahiert und der Extrakt eingedampft. Man löst 8,1 g des Rückstands in 175 ml Aethanol unter Erwärmen und gibt dazu 1,5 g Methansulfonsäure. Der erhaltene Niederschlag wird aus wässerigem Aethanol umkristallisiert. Man erhält das 1-[1-(4-Amino-6,7-dimethoxy-2-chinazolinyl)-4-piperidyl]-3-(p-methoxyphenyl)-pyrrolidin-2,5-dion-monomethansulfonat, welches bei 189-192° schmilzt.

In analoger Weise (oder gemäss Beispiel 2) wird das 1-[1-(4-Amino-6,7-dimethoxy-2-chinazolinyl)-4-piperidyl]-3-(3,4-dimethoxyphenyl)-pyrrolidin-2,5-dion-monomethansulfonat, welches bei 180-182° schmilzt, erhalten.

Der Ausgangsstoff wird wie folgt hergestellt: Ein Gemisch von 57,9 g 2-(p-Methoxyphenyl)-bernsteinsäure-anhydrid, 26,3 g 4-Amino-pyridin und 500 ml Xylol wird unter Verwendung eines Wasserabscheiders unter Rühren 6,5 Stunden unter Rückfluss gekocht. Die heisse Lösung wird dekantiert, abgekühlt, filtriert und der Rückstand aus Aethanol umkristallisiert. Man erhält das 3-(p-Methoxyphenyl)-1-(4-pyridyl)-pyrrolidin-2,5-dion, welches bei 179-180° schmilzt. Die analoge 3,4-Dimethoxy-Verbindung schmilzt bei 172-174°.

Ein Gemisch von 36 g der p-Methoxy-Verbindung, 22 g 10%-igem Palladium-auf-Kohle Katalysator und 400 ml Eisessig wird bei 100° und 1,7 Atmosphären bis zur Aufnahme der theoretischen Wasserstoffmenge hydriert. Das Reaktionsgemisch wird abgekühlt, filtriert und eingedampft. Der Rückstand wird in 100 ml Wasser gelöst, die Lösung mit Natriumcarbonat basisch gemacht, mit Methylenchlorid extrahiert und der Extrakt eingedampft. Man erhält das 3-(p-Methoxyphenyl)-1-(4-piperidyl)-pyrrolidin-2,5-dion, dessen Hydrochlorid bei 244-246° schmilzt. Das Hydrochlorid der analogen 3,4-Dimethoxy-Verbindung schmilzt bei 238-240°.

<u>Beispiel 12</u> Ein Gemisch von 6 g 3-Methyl-3-phenyl-1-(4-piperidyl)-pyrrolidin-2,5-dion, 4,79 g 4-Amino-2-chlor-6,7-dimethoxy-chinazolin und 200 ml Isoamylalkohol wird 9 Stunden unter Rückfluss gekocht, abgekühlt und mit wasserfreiem Chlorwasserstoff in Essigsäureäthylester angesäuert. Das Reaktionsgemisch wird filtriert, der Rückstand mit Isoamylalkohol und Diäthyläther gewaschen, mit 95%-igem wässerigem Aethanol trituriert und in wässeriger Natriumcarbonatlösung aufgenommen. Die Lösung wird mit Essigsäureäthylester extrahiert, der Extrakt eingedampft und der Rückstand in 100 ml Aethanol aufgenommen. Die Lösung wird mit 1,6 g Methansulfonsäure versetzt, der erhaltene Niederschlag abfiltriert und aus wässerigem Aethanol umkristallisiert. Man erhält das 1-[1-(4-Amino-6,7-dimethoxy-2-chinazolinyl)-4-piperidyl]-3-methyl-3-phenyl-pyrrolidin-2,5-dion-monomethansulfonat, welches bei 286-288° schmilzt.

Der Ausgangsstoff wird wie folgt hergestellt: Ein Gemisch von 68,6 g 2-Methyl-2-phenyl-bernsteinsäure-anhydrid, 34 g 4-Amino-pyridin und 675 ml Xylol wird unter Verwendung eines Wasserabscheiders gerührt und 7 Stunden unter Rückfluss gekocht. Das Reaktionsgemisch wird auf 40° abgekühlt, filtriert, auf 0° gekühlt und wieder filtriert. Der zweite Rückstand wird aus Isopropanol-Hexan umkristallisiert. Man erhält das 3-Methyl-3-phenyl-1-(4-pyridiyl)-pyrrolidin-2,5-dion, welches bei 90-92° schmilzt.

Ein Gemisch von 57,8 g der letztgenannten Verbindung, 22,5 g 10%-igem Palladium-auf-Kohle Katalysator und 500 ml Eisessig wird bei 120° und ungefähr 1,7 Atmosphären hydriert. Das Reaktionsgemisch wird gekühlt, filtriert und der Rückstand in wässeriger Natriumcarbonatlösung und Essigsäureäthylester aufgenommen. Die organische Lösung wird mit Wasser gewaschen, getrocknet und eingedampft. Man erhält das 3-Methyl-3-phenyl-1-(4-piperidyl)-pyrrolidin-2,5-dion,dessen Hydrochlorid, nach Umkristallisation aus Aethanol, bei 212-214° schmilzt.

Beispiel 13 Ein Gemisch von 6,16 g 3-Methyl-1-(4-piperidyl)-pyrrolidin-2,5-dion, 6,71 g 4-Amino-2-chlor-6,7-dimethoxy-chinazolin und 125 ml Isoamylalkohol wird unter Rühren 18 Stunden unter Rückfluss gekocht. Das Reaktionsgemisch wird abgekühlt, filtriert, das feste Material mit Isoamylalkohol und Diäthyläther gewaschen, mit heissem 90%-igem wässerigem Aethanol trituriert und nach Abkühlen das kristalline Produkt abfiltriert. Dieses wird in 10%-iger wässeriger Natriumcarbonatlösung gelöst und die Lösung mit Essigsäureäthylester extrahiert. Der Extrakt wird mit wässeriger Natriumcarbonatlösung und Wasser gewaschen, getrocknet und eingedampft. Der Rückstand wird in 100 ml wasserfreiem Aethanol unter Erwärmen gelöst, mit 2,14 g Methansulfonsäure versetzt und gekühlt. Das erhaltene Produkt wird abfiltriert und aus 66%-igem wässerigem Aethanol umkristallisiert. Man erhält das 1-[1-(4-Amino-6,7-dimethoxy-2-chinazolinyl)-4-piperidyl]-3-methyl-pyrrolidin-2,5-dion-monomethansulfonat, welches unter

Zersetzung bei 325-326° schmilzt.

Der Ausgangsstoff wird wie folgt hergestellt: Ein Gemisch von 28,5 g 2-Methyl-bernsteinsäure-anhydrid, 23,5 g 4-Amino-pyridin und 350 ml Xylol wird unter Verwendung eines Wasserabscheiders 3,5 Stunden gerührt und unter Rückfluss gekocht. Die heisse Lösung wird von etwas gummiartigem Rückstand dekantiert, abgekühlt und filtriert. Der Rückstand wird in 100 ml siedendem Isopropanol suspendiert und wieder filtriert. Man erhält das 3-Methyl-1-(4-pyridyl)-pyrrolidin-2,5-dion, welches bei 119-120° schmilzt.

Ein Gemisch von 34,6 g der letztgenannten Verbindung, 9 g 10%-igem Palladium-auf-Kohle Katalysator und 400 ml Eisessig wird bei 110° und ungefähr 1,7 Atmosphären bis zur Aufnahme der theoretischen Wasserstoffmenge hydriert. Das Reaktionsgemisch wird filtriert, eingedampft und der Rückstand in Wasser aufgenommen. Der pH-Wert des Gemisches wird mit Natriumcarbonat zwischen 9 und 10 eingestellt und die freigesetzte Base mit Methylenchlorid extrahiert. Der Extrakt wird getrocknet und eingedampft. Man erhält das 3-Methyl-1-(4-piperidyl)-pyrrolidin-2,5-dion, welches ohne weitere Reinigung verwendet wird.

Beispiel 14 Ein Gemisch von 4,6 g 4-Phenyl-1-(4-piperidyl)-piperidin-2,6-dion, 3,64 g 4-Amino-2-chlor-6,7-dimethoxy-chinazolin und 100 ml Isoamylalkohol wird unter Rühren 22 Stunden unter Rückfluss gekocht. Das Reaktionsgemisch wird gekühlt und mit wasserfreiem Chlorwasserstoff in Essigsäureäthylester angesäuert. Das erhaltene feste Material wird abfiltriert und mit Isoamylalkohol und Diäthyläther gewaschen. Das Produkt wird mit 90%-igem wässerigem Aethanol trituriert und dann in wässeriger Natriumcarbonatlösung aufgenommen. Das Gemisch wird mit Chloroform extrahiert und der Extrakt eingedampft. Der Rückstand wird in 75 ml Aethanol aufgenommen und mit 0,58 g Methansulfonsäure versetzt. Die erhaltene Suspension wird filtriert und der Rückstand aus wässerigem Aethanol umkristallisiert.

Man erhält das 1-[1-(4-Amino-6,7-dimethoxy-2-chinazolinyl)-4-piperidyl]-4-phenyl-piperidin-2,6-dion-monomethansulfonat, welches unter Zersetzung bei 292-295° schmilzt.

In analoger Weise wird auch das 1-[1-(4-Amino-6,7-dimethoxy-2-chinazolinyl)-4-piperidyl]-4-(p-methoxy-phenyl)-piperidin-2,6-dion-monomethansulfonat, welches unter Zersetzung bei 305-307° schmilzt, erhalten.

Der Ausgangsstoff wird wie folgt hergestellt: Ein Gemisch von 19 g 3-Phenylglutarsäure-anhydrid, 10,3 g 4-Amino-pyridin, 350 ml Xylol und 2,5 g Methansulfonsäure wird unter Verwendung eines Wasserabscheiders unter Rühren 2 Tage unter Rückfluss gekocht. Die heisse Lösung wird von etwas unlöslichem Material dekantiert, gekühlt und filtriert. Der Rückstand wird mit heissem Isopropanol trituriert. Man erhält das 4-Phenyl-1-(4-pyridyl)-piperidin-2,6-dion, welches bei 229-230° schmilzt. Das in analoger Weise hergestellte 4-(p-Methoxy-phenyl)-1-(4-pyridyl)-piperidin-2,6-dion schmilzt bei 168-169°.

Ein Gemisch von 14,6 g 4-Phenyl-1-(4-pyridyl)-piperidin-2,6-dion, 12,3 g 10%-igem Palladium-auf-Kohle Katalysator und 200 ml Eisessig wird bei 100° und 1,7 Atmosphären 8 Stunden hydriert. Nach Abkühlen wird das Reaktionsgemisch filtriert, eingedampft, der Rückstand in einer minimalen Menge Wasser gelöst und die Lösung mit Natriumcarbonat basisch gestellt. Die Lösung wird mit Essigsäureäthylester extrahiert und der Extrakt eingedampft. Der Rückstand wird durch Trituieren mit 75 ml Diäthyläther-Hexan (3:1) kristallisiert und aus 50 ml Isopropanol umkristallisiert. Man erhält das 4-Phenyl-1-(4-piperidyl)-piperidin-2,6-dion, welches bei 182-189° schmilzt. Die analoge p-Methoxy-phenyl-Verbindung schmilzt bei 127-129°.

<u>Beispiel 15</u> Herstellung von 10'000 Tabletten mit einem Gehalt von je 2,5 mg der aktiven Substanz:

Bestandteile:

| | |
|---|---|
| 1-[1-(4-Amino-6,7-dimethoxy-2-chinazolinyl)-4-piperidyl]-pyrrolidin-2,5-dion-monomethansulfonat | 25 g |
| Milchzucker | 1'956 g |
| Maisstärke | 90 g |
| Polyäthylenglykol 6000 | 90 g |
| Talkpulver | 90 g |
| Magnesiumstearat | 24 g |
| Gereinigtes Wasser | q.s. |

Verfahren:

Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Särke in einem geeigneten Mischer vermischt. Die andere Hälfte der Stärke wird in 45 ml Wasser suspendiert und die Suspension zur siedenden Lösung von Polyäthylenglykol in 180 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten von 7,1 mm Durchmesser, welche eine Bruchrille aufweisen, gepresst.

Herstellung von 10'000 Kapseln mit einem Gehalt von je 5 mg der oben genannten aktiven Substanz:

Bestandteile:

| | |
|---|---|
| Das genannte Monomethansulfonat | 50 g |
| Milchzucker | 2'350 g |
| Talkpulver | 150 g |

Verfahren:

Sämtliche pulverigen Bestandteile werden mit einem Sieb von 0,6 mm Maschenweite gesiebt. Dann wird der Wirkstoff zuerst mit Talk und darauffolgend mit dem Milchzucker in einem geeigneten Mischer homogenisiert. Kapseln Nr. 2 werden mit je 300 mg des Gemisches mit einer Füllmaschine gefüllt.

In analoger Weise werden Tabletten oder Kapseln, welche eine andere Verbindung der Erfindung, z.B. eine solche der vorhergehenden Beispiele enthalten, hergestellt.

Beispiel 16 Ein Gemisch von 4,79 g 4-Amino-2-chlor-6,7-dimethoxy-chinazolin, 5,2 g 2-(4-Piperidyl)-hexahydroisoindol-1,3-dion und 100 ml Isoamylalkohol wird in einer Stickstoffatmosphäre, unter Rühren, 20 Stunden unter Rückfluss gekocht. Das Reaktionsgemisch wird abgekühlt und mit einer Lösung von wasserfreiem Chlorwasserstoff in Essigsäureäthylester angesäuert. Der erhaltene Niederschlag wird abfiltriert, mit Aethanol gewaschen, mit wässeriger Natriumcarbonatlösung trituriert und mit Essigsäureäthylester extrahiert. Der Extrakt wird mit Wasser gewaschen, getrocknet und eingedampft. Der Rückstand wird in wasserfreiem Aethanol gelöst, die Lösung mit Methansulfonsäure neutralisiert, der erhaltene Niederschlag abfiltriert und aus wässerigem Aethanol umkristallisiert. Man erhält das 2-[1-(4-Amino-6,7-dimethoxy-2-chinazolinyl)-4-piperidyl]-hexahydroisoindol-1,3-dion-monomethansulfonat, welches bei 303-305° schmilzt.

Der Ausgangsstoff wird wie folgt hergestellt: Ein Gemisch von 23,5 g 4-Amino-pyridin, 38,5 g cis-1,2-Cyclohexan-dicarbonsäure-anhydrid und 400 ml Xylol wird gerührt und unter Verwendung eines Wasserabscheiders 42 Stunden unter Rückfluss gekocht. Nach der ersten halben Stunde und nach 18 Stunden wird das Reaktionsgemisch mit jeweils 1 ml Methansulfonsäure versetzt. Das heisse Reaktionsgemisch wird filtriert. Die beim Abkühlen erhaltenen Kristalle werden abfiltriert

und aus Isopropanol umkristallisiert. Man erhält das 2-(4-Pyridyl)-hexahydroisoindol-1,3-dion, welches bei 165-168° schmilzt.

Ein Gemisch von 38,7 g der letztgenannten Verbindung, 10 g 5%igem Rhodium-Katalysator auf Aluminiumoxyd und 400 ml Eisessig wird bei 75-105° drei Stunden bei 3 Atmosphären hydriert. Nach Abkühlen wird das Reaktionsgemisch filtriert, eingedampft, der Rückstand mit gesättigter wässeriger Natriumcarbonatlösung behandelt und mit Methylenchlorid extrahiert. Der Extrakt wird mit Wasser gewaschen und eingedampft. Man erhält das 2-(4-Piperidyl)-hexahydroisoindol-1,3-dion. Sein Hydrochlorid schmilzt bei 241-243°.

Beispiel 17 Ein Gemisch von 2,2 g 8-Amino-6-chlor-1,3-dioxolo[4,5-g]-chinazolin, 2,2 g 1-(4-Piperidyl)-pyrrolidin-2,5-dion und 60 ml Isoamylalkohol wird unter Rühren 20 Stunden unter Rückfluss gekocht. Das Reaktionsgemisch wird gekühlt, filtriert, das erhaltene feste Material mit Isoamylalkohol und Diäthyläther gewaschen und getrocknet. Das Produkt wird mit 10%iger wässeriger Natriumcarbonatlösung trituriert und mit Essigsäureäthylester extrahiert. Der Extrakt wird mit wässeriger Natriumcarbonatlösung und Wasser gewaschen, getrocknet und eingedampft. Der Rückstand wird unter Erwärmen in wasserfreiem Aethanol gelöst und mit Methansulfonsäure neutralisiert. Nach Abkühlen wird das Produkt abfiltriert und aus wässerigem Aethanol umkristallisiert. Man erhält das 1-[1-(8-Amino-1,3-dioxolo[4,5-g]-6-chinazolinyl)-4-piperidyl]-pyrrolidin-2,5-dion-monomethansulfonat, welches bei 348-350° unter Zersetzung schmilzt.

In analoger Weise, vorzugsweise gemäss den Beispielen 1, 12 und 13 werden auch das 1-[1-(4-Amino-6,7-dimethoxy-2-chinazolinyl)-4-piperidyl]-3,3-dimethyl-pyrrolidin-2,5-dion-monomethan-sulfonat (F. 267-268°) und das 1-[1-(4-Amino-6,7-dimethoxy-2-chinazolinyl)-4-piperidyl]-3,4-dimethyl-pyrrolidin-2,5-dion-monomethansulfonat (F. 314-315°) hergestellt.

Patentansprüche

(für alle benannten Länder ausser Oesterreich)

1. N-[1-(4-Amino-2-chinazolinyl)-3- oder -4-piperidyl-lactame der allgemeinen Formel I

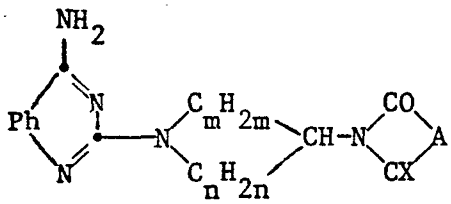

(I),

worin Ph einen 1,2-Phenylenrest bedeutet, der gegebenenfalls durch höchstens 3 Substituenten ausgewählt von Niederalkyl und Niederalkoxy und einem Niederalkylendioxy substituiert sein kann, jedes der Symbole m und n für eine ganze Zahl von 1 bis 3 steht, wobei m + n = 4, X 2 Wasserstoffatome oder Oxo bedeutet, und A für Niederalkylen, 4 bis 7 Ringglieder enthaltendes Cycloalkylen, Cycloalkylniederalkylen und Spirocycloalkan-niederalkylen, HPh-Niederalkylen oder Ph steht, und ihre Säureadditionssalze.

2. Verbindungen der im Anspruch 1 gezeigten Formel I, worin Ph einen 1,2-Phenylenrest bedeutet, der gegebenenfalls durch höchstens 3 Substituenten ausgewählt von Alkyl oder Alkoxy mit höchstens 4 Kohlenstoffatomen und einem Alkylendioxy mit höchstens 2 Kohlenstoffatomen substituiert sein kann, jedes der Symbole m und n für eine ganze Zahl von 1 bis 3 steht, wobei m + n = 4, X zwei Wasserstoffatome oder Oxo bedeutet, und A für Niederalkylen, 4 bis 7 Ringglieder enthaltendes Cycloalkylen, Cycloalkyl-niederalkylen, Spirocycloalkan-niederalkylen, HPh-Niederalkylen oder Ph steht, und ihre therapeutisch verwendbaren Säureadditionssalze.

3. Verbindungen der im Anspruch 1 gezeigten Formel I, worin Ph 1,2-Phenylen, Mono-, Di- oder Tri-(alkyl oder alkoxy)-1,2-phenylen oder Alkylendioxy-1,2-phenylen bedeutet, in welchen Alkyl, Alkoxy oder Alkylen höchstens 2 Kohlenstoffatome enthält, jede der Gruppen $C_mH_{2m}$ und $C_nH_{2n}$ für Aethylen steht, X zwei Wasserstoffatome oder Oxo bedeutet, und A für Niederalkylen, 5 oder 6 Ringglieder enthaltendes 1,2-Cycloalkylen oder (Cycloalkyl, Spirocycloalkan oder Phenyl)-alkylen steht, wobei jeder der letztgenannten drei Reste eine Summe von höchstens 8 Kohlenstoffatomen enthält, und ihre therapeutisch verwendbaren Säureadditionssalze.

4. Verbindungen der allgemeinen Formel II

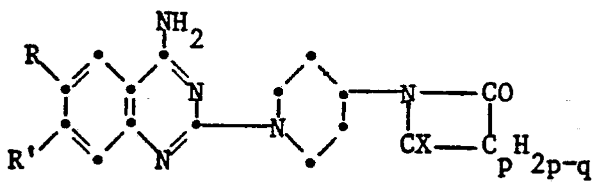

(II),

worin jedes der Symbole R und R' Methoxy oder zusammen Methylendioxy bedeutet, X für zwei Wasserstoffatome oder Oxo steht, $C_pH_{2p-q}$ Alkylen, Phenyl-alkylen oder Spirocycloalkan-alkylen bedeutet, wobei p eine ganze Zahl von 2 bis 8 ist, q die Zahl 0,2 oder 8 bedeutet, und $2p-q$ positiv ist, und ihre therapeutisch verwendbaren Säureadditionssalze.

5. Verbindungen der im Anspruch 4 gezeigten Formel II, worin jedes der Symbole R und R' Methoxy bedeutet, X für Oxo steht und das Symbol $C_pH_{2p-q}$ Aethylen, Phenyläthylen oder 1,3-Propylen bedeutet, und ihre therapeutisch verwendbaren Säureadditionssalze.

6. Eine Verbindung der Gruppe bestehend aus

1-[1-(4-Amino-6,7-dimethoxy-2-chinazolinyl)-4-piperidyl]-piperidin-2,6-dion,

1-[1-(4-Amino-6,7-dimethoxy-2-chinazolinyl)-4-piperidyl]-4,4-dimethyl-piperidin-2,6-dion,

8-[1-(4-Amino-6,7-dimethoxy-2-chinazolinyl)-4-piperidyl]-8-azaspiro-[4,5]decan-7,9-dion,

8-[1-(4-Amino-6,7,8-trimethoxy-2-chinazolinyl)-4-piperidyl]-8-aza-spiro[4,5]decan-7,9-dion,

3-[1-(4-Amino-6,7-dimethoxy-2-chinazolinyl)-4-piperidyl]-3-azaspiro-[5,5]undecan-2,4-dion,

2-[1-(4-Amino-6,7-dimethoxy-2-chinazolinyl)-4-piperidyl]-2-azaspiro-[4,4]nonan-1,3-dion,

1-[1-(4-Amino-6,7-dimethoxy-2-chinazolinyl)-4-piperidyl]-3-phenyl-pyrrolidin-2,5-dion,

2-[1-(4-Amino-6,7,8-trimethoxy-2-chinazolinyl)-4-piperidyl]-isoindolin-1-on,

2-[1-(4-Amino-6,7-dimethoxy-2-chinazolinyl)-4-piperidyl]-isoindolin-1-on,

1-[1-(4-Amino-6,7-dimethoxy-2-chinazolinyl)-4-piperidyl]-4-cyclohexyl-piperidin-2,6-dion,

1-[1-(4-Amino-6,7-dimethoxy-2-chinazolinyl)-4-piperidyl]-3-(p-methoxy-phenyl)-pyrrolidin-2,5-dion,

1-[1-(4-Amino-6,7-dimethoxy-2-chinazolinyl)-4-piperidyl]-3-(3,4-dimethoxyphenyl)-pyrrolidin-2,5-dion,

1-[1-(4-Amino-6,7-dimethoxy-2-chinazolinyl)-4-piperidyl]-3-methyl-3-phenyl-pyrrolidin-2,5-dion,

1-[1-(4-Amino-6,7-dimethoxy-2-chinazolinyl)-4-piperidyl]-3-methyl-pyrrolidin-2,5-dion,

1-[1-(4-Amino-6,7-dimethoxy-2-chinazolinyl)-4-piperidyl]-4-phenyl-piperidin-2,6-dion,

1-[1-(4-Amino-6,7-dimethoxy-2-chinazolinyl)-4-piperidyl]-4-(p-methoxy-phenyl)-piperidin-2,6-dion,

2-[1-(4-Amino-6,7-dimethoxy-2-chinazolinyl]-4-piperidyl]-hexahydro-isoindol-1,3-dion,

1-[1-(8-Amino-1,3-dioxolo[4,5-g]-6-chinazolinyl)-4-piperidyl]-pyrrolidin-2,5-dion,

1-[1-(4-Amino-6,7-dimethoxy-2-chinazolinyl)-4-piperidyl]-3,3-dimethyl-pyrrolidin-2,5-dion,

1-[1-(4-Amino-6,7-dimethoxy-2-chinazolinyl)-4-piperidyl]-3,4-dimethyl-pyrrolidin-2,5-dion und den therapeutisch verwendbaren Säureadditionssalzen dieser Verbindungen.

7. 1-[1-(4-Amino-6,7-dimethoxy-2-chinazolinyl)-4-piperidyl)-pyrrolidin-2,5-dion und seine therapeutisch verwendbaren Säureadditionssalze.

8. Pharmazeutische Präparate enthaltend eine Verbindung gemäss einem der Ansprüche 1 bis 7.

9. Die Verbindungen der Ansprüche 1 bis 7 zur Verwendung als Pharmazeutika.

10. Verfahren zur Herstellung von neuen N-[1-(4-Amino-2-chinazolinyl)-3- oder -4-piperidyl-lactamen der allgemeinen Formel I

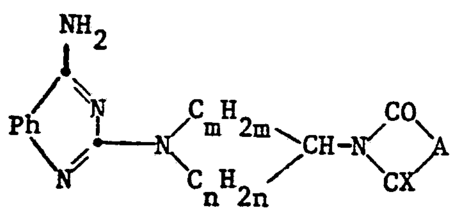

(I),

worin Ph einen 1,2-Phenylenrest bedeutet, der gegebenenfalls durch höchstens 3 Substituenten ausgewählt von Niederalkyl und Niederalkoxy und einem Niederalkylendioxy substituiert sein kann, jedes der Symbole m und n für eine ganze Zahl von 1 bis 3 steht, wobei $m + n = 4$, X 2 Wasserstoffatome oder Oxo bedeutet, und A für Niederalkylen, 4 bis 7 Ringglieder enthaltendes Cycloalkylen, Cycloalkylniederalkylen und Spirocycloalkan-niederalkylen, HPh-Niederalkylen oder Ph steht, und ihren Säureadditionssalzen, dadurch gekennzeichnet, dass man Verbindungen der Formeln III und IV

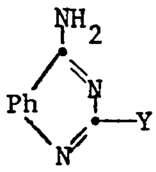 + 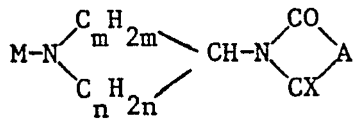

(III) (IV)

kondensiert, worin Y Halogen oder Niederalkythio bedeutet und M für Wasserstoff oder ein Alkalimetallatom steht, und, wenn erwünscht, eine erhaltene Verbindung der Formel I in eine andere erfindungsgemässe Verbindung umwandelt, und/oder, wenn erwünscht, eine erhaltene freie Verbindung in ein Säureadditionssalz oder ein Säureadditionssalz in die freie Verbindung oder in ein anderes Salz überführt, und/oder, wenn erwünscht, ein erhaltenes Gemisch von Isomeren oder Razematen in die einzelnen Isomeren oder Razemate auftrennt, und/oder, wenn erwünscht, erhaltene Ratemate in die optischen Antipoden aufspaltet.

Patentansprüche

(fUr Oesterreich)

1. Verfahren zur Herstellung von neuen N-[1-(4-Amino-2-chinazolinyl)-3- oder -4-piperidyl-lactamen der allgemeinen Formel I

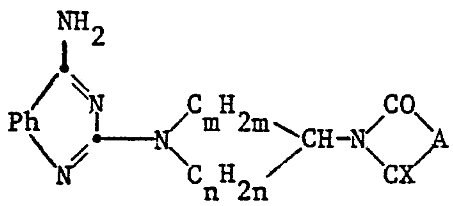

(I),

worin Ph einen 1,2-Phenylenrest bedeutet, der gegebenenfalls durch höchstens 3 Substituenten ausgewählt von Niederalkyl und Niederalkoxy und einem Niederalkylendioxy substituiert sein kann, jedes der Symbole m und n für eine ganze Zahl von 1 bis 3 steht, wobei m + n = 4, X 2 Wasserstoffatome oder Oxo bedeutet, und A für Niederalkylen, 4 bis 7 Ringglieder enthaltendes Cycloalkylen, Cycloalkylniederalkylen und Spirocycloalkan-niederalkylen, HPh-Niederalkylen oder Ph steht, und ihren Säureadditionssalzen, dadurch gekennzeichnet, dass man Verbindungen der Formeln III und IV

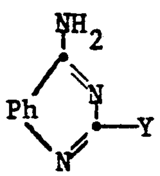

+

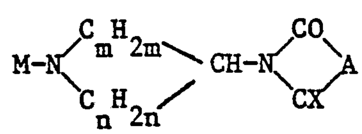

(III) (IV)

kondensiert, worin Y Halogen oder Niederalkythio bedeutet und M für Wasserstoff oder ein Alkalimetallatom steht, und, wenn erwünscht, eine erhaltene Verbindung der Formel I in eine andere erfindungsgemässe Verbindung umwandelt , und/oder, wenn erwünscht, eine erhaltene freie Verbindung in ein Säureadditionssalz oder ein Säureadditionssalz in die freie Verbindung oder in ein anderes Salz überführt, und/oder, wenn erwünscht, ein erhaltenes Gemisch von Isomeren oder Razematen in die einzelnen Isomeren oder Razemate auftrennt, und/

oder, wenn erwünscht, erhaltene Razemate in die optischen Antipoden aufspaltet.

2. Verfahren nach Anspruch 1, dadurch gekennzeichnet, dass man Ausgangsstoffe der Formeln III und IV verwendet, in welchen Y Chlor, Brom oder Methylthio bedeutet, und M für Natrium oder Kalium steht.

3. Verfahren nach einem der Ansprüche 1 und 2, dadurch gekennzeichnet, dass man Verbindungen der allgemeinen Formel I, worin Ph einen 1,2-Phenylenrest bedeutet, der gegebenenfalls durch höchstens 3 Substituenten ausgewählt von Alkyl oder Alkoxy mit höchstens 4 Kohlenstoffatomen und einem Alkylendioxy mit höchstens 2 Kohlenstoffatomen substituiert sein kann, jedes der Symbole m und n für eine ganze Zahl von 1 bis 3 steht, wobei m + n = 4, X zwei Wasserstoffatome oder Oxo bedeutet, und A für Niederalkylen, 4 bis 7 Ringglieder enthaltendes Cycloalkylen, Cycloalkyl-niederalkylen, Spirocycloalkan-niederalkylen, HPh-Niederalkylen oder Ph steht, und ihre Säureadditionssalze herstellt.

4. Verfahren nach einem der Ansprüche 1 und 2, dadurch gekennzeichnet, dass man Verbindungen der allgemeinen Formel I, worin Ph 1,2-Phenylen, Mono-, Di- oder Tri-(alkyl oder alkoxy)-1,2-phenylen oder Alkylendioxy-1,2-phenylen bedeutet, in welchen Alkyl, Alkoxy oder Alkylen höchstens 2 Kohlenstoffatome enthält, jede der Gruppen $C_mH_{2m}$ und $C_nH_{2n}$ für Aethylen steht, X zwei Wasserstoffatome oder Oxo bedeutet, und A für Niederalkylen, 5 oder 6 Ringglieder enthaltendes 1,2-Cycloalkylen oder (Cycloalkyl, Spirocycloalkan oder Phenyl)-alkylen steht, wobei jeder der letztgenannten drei Reste eine Summe von höchstens 8 Kohlenstoffatomen enthält, und ihre Säureadditionssalze herstellt.

5. Verfahren nach einem der Ansprüche 1 und 2, dadurch gekennzeichnet, dass man Verbindungen der allgemeinen Formel II

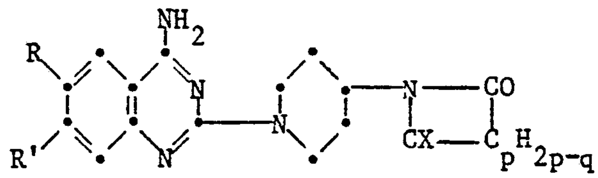

(II),

worin jedes der Symbole R und R' Methoxy oder zusammen Methylendioxy bedeutet, X für zwei Wasserstoffatome oder Oxo steht, $C_pH_{2p-q}$ Alkylen, Phenyl-alkylen oder Spirocycloalkan-alkylen bedeutet, wobei p eine ganze Zahl von 2 bis 8 ist, q die Zahl 0,2 oder 8 bedeutet, und $2p-q$ positiv ist, und ihre Säureadditionssalze herstellt.

6. Verfahren nach einem der Ansprüche 1 und 2, dadurch gekennzeichnet, dass man Verbindungen der allgemeinen Formel II, worin jedes der Symbole R und R' Methoxy bedeutet, X für Oxo steht, und das Symbol $C_pH_{2p-q}$ Aethylen, Phenyläthylen oder 1,3-Propylen bedeutet, und ihre Säureadditionssalze herstellt.

7. Verfahren nach einem der Ansprüche 1 und 2, dadurch gekennzeichnet, dass man 1-[1-(4-Amino-6,7-dimethoxy-2-chinazolinyl)-4-piperidyl]-pyrrolidin-2,5-dion und seine Säureadditionssalze herstellt.

8. Verfahren zur Herstellung eines pharmazeutischen Präparates, gekennzeichnet, durch die Verarbeitung eines der erfindungsgemässen Wirkstoffe mit einem pharmazeutischen Trägermaterial.

Europäisches Patentamt

# EUROPÄISCHER RECHERCHENBERICHT

0009465

Nummer der Anmeldung
EP 79 81 0094

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | FR - A - 2 321 890 (SYNTHELABO)<br>* Seiten 1-4, 11-15 *<br>---- | 1,8,10 | A 61 K 31/00<br>C 07 D 401/14//<br>C 07 D 401/04 |

RECHERCHIERTE SACHGEBIETE (Int. Cl. 3)

C 07 D 401/04
401/14

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 18-12-1979 | FRANCOIS |